Europäisches Patentamt

European Patent Office

Office européen des brevets

(19)

(11) Publication number : **0 391 725 B1**

# EUROPEAN PATENT SPECIFICATION

(12)

(45) Date of publication of patent specification :
22.06.94 Bulletin 94/25

(51) Int. Cl.⁵ : **A61B 19/00, A41D 13/00**

(21) Application number : 90303701.8

(22) Date of filing : 06.04.90

(54) Method for making an electrostatically charged face mask.

(30) Priority : 07.04.89 US 335157

(43) Date of publication of application :
10.10.90 Bulletin 90/41

(45) Publication of the grant of the patent :
22.06.94 Bulletin 94/25

(84) Designated Contracting States :
AT BE CH DE ES FR GB IT LI LU NL SE

(56) References cited :
WO-A-81/03266
FR-A- 2 416 535
GB-A- 2 014 059
US-A- 4 375 718

(73) Proprietor : JOHNSON & JOHNSON MEDICAL,
INC.
One Johnson & Johnson Plaza
New Brunswick, New Jersey 08933 (US)

(72) Inventor : Singer, Wayne Jay
6711 Potomac Parkway
Arlington, Texas 76017 (US)

(74) Representative : Mercer, Christopher Paul et al
Carpmaels & Ransford
43, Bloomsbury Square
London WC1A 2RA (GB)

## Description

### Field of the Invention

The invention relates to a production method for face masks for surgical and medical use. More particularly, the present invention relates to production of face masks wherein the filtration medium comprises at least two individual layers of filtration medium in face-to-face contact and wherein said filtration medium, together with the inner and outer facings thereof, are electrostatically charged. The face masks produced by the method of the invention have good blood repellency and are highly efficient in filtering out bacteria and particulate matter having sizes ranging to as low as 0.1 micron in diameter. The mask is particularly suitable for use by surgeons and other operating room personnel during the performance of laser surgery.

### Background of the Invention

Surgical face masks are routinely employed by the operating staff during the course of surgical procedures. The purpose of the face mask has traditionally been to prevent bacteria exhaled by the surgeon, or other members of the operating room staff, from contaminating the patient undergoing surgery. These face masks comprise a filtration medium having first and second major surfaces, an inner facing layer which covers one major surface of the filtration medium and an outer facing layer which covers the other major surface of the filtration medium. In the finished face mask, the filtration medium and the inner and outer facing layers are coextensive in length and width. The face masks are usually provided with bindings along their top and bottom edges and their side edges. In some instances, the top and bottom edges are extended to provide tie strings, while in other cases the side bindings are extended for the same purpose. In addition, face masks often are provided with a "nose clip" along or adjacent their upper edge. This nose clip comprises a piece of malleable material such as aluminum which can be deformed to fit across the bridge of the wearer's nose and help keep the mask in place.

The filtration medium has a pore size which is sufficiently small to prevent bacteria from flowing through the mask. Bacteria, in general, have diameters on the order of from 1-5 microns. Heretofore, fiberglass mats were used as the filter medium but more recently the filter medium has comprised a web of generally discontinuous, small diameter polypropylene fibers made by what is known in the industry as a melt blowing process.

In recent times lasers have been employed in surgery to precisely excise tissue, e.g. a tumor, by vaporizing it, a process sometimes referred to as "photoablation". The photoablation process produces "smoke" or "plume" which comprises not only water vapor but also particulate matter, e.g. tissue fragments, whose diameter is considerably smaller than that of bacteria. It is known that those tissue fragments may carry intact viruses and so it has been suggested that laser plume be regarded as potentially infectious. In view of the foregoing, it is desirable that the filtration medium used in surgical face masks be capable of filtering out particles whose diameters are considerably smaller than the diameters of bacteria and viruses. In this connection, it would be desirable to provide a production method for a face mask whose filtration medium would be able to efficiently filter particles having diameters on the order of 0.1 micron.

It is also possible that blood from a surgical patient may be projected upwardly toward the surgeon and other personnel during the course of a surgical procedure. It is therefore desirable that surgical face masks be repellent to blood so that the facial area is protected from potentially harmful contamination.

US-A-4375718 discloses a process of manufacturing an electrostatically charged filtration medium. A web made of nonconductive thermoplastic fibers is contacted on each side with a more conductive web to form a combined web. The combined web is charged with electrically charged particles from corona charging elements on opposite sides of the web. The charging elements are operated at a voltage of from 5 to 25 kV but with opposite polarity.

US-A-4215682 discloses the introduction of a persistent electric charge into melt-blown fibers during the melt-blowing process. When these charged fibers are incorporated into fibrous webs, they provide unique properties, including improved filtering properties.

### Summary of the Invention

In accordance with the present invention, there is provided a method as defined in claim 1 for making an electrostatically charged body portion of a face mask. According to embodiments of this method, a first layer of melt blown fibers is deposited on the first facing layer, after which the resulting two-ply laminate is electrostatically charged at 15-20 kilovolts (kv) in an electrostatic charging unit. The second layer of melt blown fibers is then deposited on the second facing layer to give a second two-ply laminate. The first two-ply laminate (which has been previously electrostatically charged), is then superposed on the second two-ply laminate (which has not been previously charged) so that the melt blown fiber layers of the first and second laminates are brought into the above-described face-to-face, contacting relationship. This results in a third laminate, which has four plies and comprises, in sequence through its thickness, a first air permeable facing layer, a first individual layer of filtration medium comprising a layer of melt blown fibers, a second individual layer of filtration medium also comprising a layer of melt blown fibers, and a second air permeable facing layer. The resulting four-ply laminate is then electrostatically charged at 15-20 kv to give an electrostatically charged body portion material which can be converted (in known fashion) to a surgical or the like face mask. It will be understood that according to the described method, the two-ply laminate comprising the first facing layer of melt blown fibers is exposed to the electrostatic charging unit on two occasions. The second two-ply laminate comprising the second facing layer and the second layer of melt blown fibers is exposed to the charging unit on one occasion.

It will be understood by those skilled in the art that the aforementioned "first facing layer' can eventually serve as either the outer facing layer or the inner, face contacting layer of the finished face mask. The aforementioned "second facing layer" would then serve, conversely, as the inner, face contacting layer or the outer facing layer, respectively, of the mask.

By the method of the present invention, there is produced a face mask having an electrostatically charged body portion comprising a first air permeable facing layer, a filtration medium comprising at least two individual, separate and distinct layers of melt blown fibers, and a second air permeable facing layer. The first and second air permeable facing layers are preferably made from a nonwoven fabric although other air permeable materials such as gauze could also be used. The melt blown fibers are preferably polypropylene fibers, but nylon fibers could also be used. The melt blown fibers may have diameters of up to about 10 microns. Preferably, however, the melt blown fibers have diameters ranging from about 2 microns to about 6 microns, and more preferably from about 2.5 microns to about 5.5 microns. Preferably, the lengths of the melt blown fibers range from 76.2 mm (3 inches) to 127 mm (5 inches).

The aforesaid individual melt blown fiber layers are superposed one on top of the other in face-to-face, contacting relationship to provide the filtration medium component of the body portion. The individual layers of melt blown fibers are held in place with respect to each other by frictional engagement of their fibers and without using additional securement means such as adhesives or ultrasonic bonding. The individual fibrous layers comprising the filtration medium of the face mask may be readily peeled apart from one another since they are held in their face-to-face, contacting relationship only by the above-mentioned frictional engagement of their fibers. When the filtration medium is used in face masks, the said individual fibrous layers, even though they may be bound together at their top, bottom and side edges, are nevertheless free to move with respect to one another in response to the inhaling of air and the exhaling of breath. As mentioned, the melt blown thermoplastic fibers may be derived from polypropylene or nylon but polypropylene is preferred because of its lower cost and because more is known about the technology used for producing polypropylene melt blown webs than that used for producing melt blown webs from nylon. The basis weight of the individual layers of melt blown fibers comprising the filtration medium may range, when intended for use in surgical face masks, from about 6.78 $gm^{-2}$ (0.2 ounce/yd$^2$) to about 27.12 $gm^{-2}$ (0.8 ounce/yd$^2$) so as to give total weights of at least 13.56 $gm^{-2}$ (0.4 ounce/yd$^2$) to 54.24 $gm^{-2}$ (1.6 ounce/yd$^2$). The individual layers may have higher basis weights when intended for use in other filtration applications. The weight of the individual melt blown fibrous layers may be the same or different but it is preferred, for ease of production, that the individual fibrous layers have substantially the same weight. For example, the filtration medium may comprise, two individual melt blown fibrous layers each having a basis weight of 20.34 $gm^{-2}$ (0.6 ounce/yd$^2$) or it may comprise, a first fibrous layer whose basis weight is 16.95 $gm^{-2}$ (0.5 ounce/yd$^2$) and a second fibrous layer whose basis weight is 23.73 $gm^{-2}$ (0.7 ounce/yd$^2$), the basis weight of the two-layer filtration medium being 40.68 $gm^{-2}$ (1.2 ounce/yd$^2$) in both embodiments.

The melt blown fibrous layerss comprising the above-described filtration medium are made, as indicated earlier herein, by a melt blowing process which is known per se in the art. In brief, melt blown fibrous webs are prepared by extruding a molten thermoplastic polymer, such as molten polypropylene, through a multi-orifice die and collecting the extruded thermoplastic filaments or strands on a foraminous collecting surface. The extrusion die is placed at a distance, e.g. 30.48 cm (12 inches), from the collecting surface and a high velocity gas, usually air, is used to attenuate the extruded molten filaments or strands exiting the die and break them into discontinuous fibers of small diameter. These discontinuous fibers, sometimes called "microfibers", are collected in the form of a fibrous layer or web which is often referred to as a "melt blown layer" or "melt blown web". The microfibers in such layers or webs have diameters ranging up to about 10 microns, with the average diameter of the fibers ranging from about 2 to about 6 microns. The microfibers are predominantly discontinuous and generally have lengths, e.g. about 76.2 to 127 mm (3 to 5 inches), which exceed those normally associated with staple fibers. A melt blowing process is disclosed in an article entitled "Superfine Thermoplastic Fibers" appearing in Industrial & Engineering Chemistry, Vol. 48, No. 8, (1956) at pages 1342-1346. Other melt blowing processes are disclosed in U.S. Patent No. 3,849,241 and the several patents and related publications mentioned therein.

Brief Description of the Drawings

The invention will be better understood with reference to the appended drawings in which:

FIGURE 1 is a plan view, with portions cut away, of one embodiment of a face mask produced by the method of the present invention;

FIGURE 2 is a schematic cross-sectional view taken along line 2-2 of FIG. 1;

FIGURE 3 is an enlarged cross-sectional view taken along line 2-2 of FIG. 1 and showing the inner facing layer, the two-layer filtration medium and the outer facing layer comprising the face mask of FIG. 1;

FIGURE 4 is an exploded cross-sectional view similar to FIG. 3;

FIGURE 5 is a side elevational view of apparatus used in the first stage of the process according to the present invention; and

FIGURE 6 is a side elevational view of apparatus used in the second stage of the process according to the present invention.

Detailed Description of the Invention

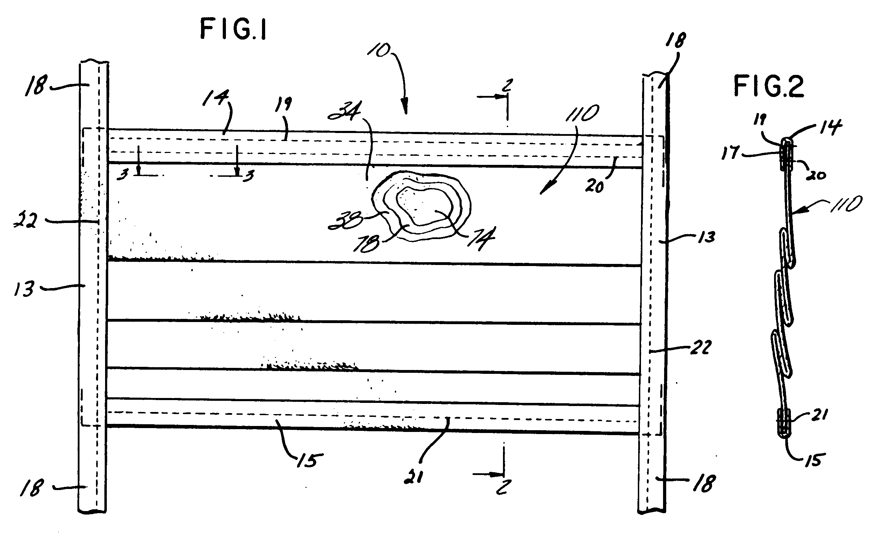

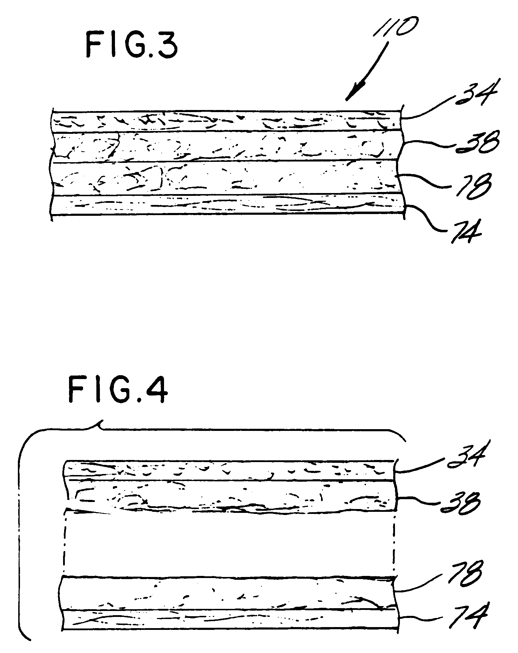

Referring now to the drawings, and especially to FIGS. 1-4 thereof, there is shown a surgical face mask 10 comprising a main body portion 110 which has a binding 14 along its upper edge and a binding 15 along its lower edge. Body portion 21 also has bindings 13,13 along the side edges thereof, which bindings may be extended at the corners of the mask, if desired, to provide tie strings 18 which may be tied at the back of the head of the wearer in order to secure the mask in its desired position. Upper binding 14 is secured on the main body portion 110 by two rows of stitching 19,20, while lower binding 15 is secured in place by stitching 21. Side bindings 13,13 are secured by stitching 22. As is well-known in the art, side bindings 13,13 and tie strings 18,18 may comprise biased fabric tapes and may, if desired, have elastic characteristics. The upper region of body portion 110 of face mask 10 may carry a nose clip 17 (see Fig. 2) which may be formed, for example, from a thin strip or wire-like element of a malleable material such as aluminum. When the mask is used, the nose clip may be bent to conform to the bridge of the wearer's nose, thus helping to keep the upper regions of the face mask in close contact with the face of the wearer. If desired, the mask may be provided with a sheet of air impervious material (not illustrated in the drawings) disposed across the upper outer surface of the mask. The presence of the thin layer of air impervious material will help prevent exhaled breath of the wearer of the mask from rising and fogging his or her eyewear. Further details about the use of such an air impervious material, along with other features of surgical face masks, are disclosed in U.S. Patent Nos. 3,888,246 and 3,890,966, the teachings of both of which patents are hereby incorporated by reference.

Main body portion 110, shown schematically in FIG. 2 of the drawings, comprises an air permeable outer facing 34, a first layer of filtration medium 38, a second layer of filtration medium 78, and an air impermeable inner facing layer 74. Facing layers 34 and 74 may be made from any highly air permeable material, nonwoven fabrics being known and especially useful for this purpose. A particularly preferred facing fabric comprises a print bonded nonwoven fabric containing a blend of 65% by weight of woodpulp and 35% by weight of rayon staple fibers. In the embodiment under discussion, filtration layers 38 and 78 preferably comprise melt blown polyester fibers. As indicated earlier herein, various methods for making such melt blown fibrous layers are well-known in the art; accordingly, such methods will not be described in detail herein.

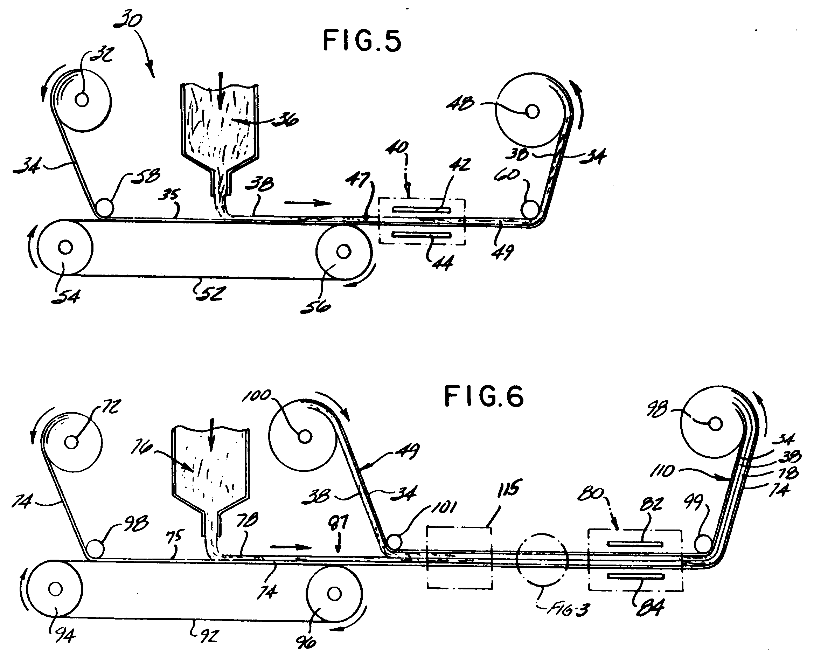

A process for making a surgical face mask in accordance with the present invention will now be described with reference to FIGS. 5 and 6 of the drawings. The apparatus 30 of FIG. 5 comprises an undriven let-off roll 32 containing a roll of a first air permeable facing fabric 34; an apparatus 36 for making a first web or layer 38 of melt blown polypropylene fibers; an electrostatic charging unit 40 (available as Simco Chargemaster from Simco, Inc., Cincinatti, Ohio) comprising an upper discharge bar 42 and a lower discharge bar 44; a driven take up roller 48 for winding up the electrostatically charged laminate 49 comprising facing fabric 34 and melt blown web 38; and a conveyor means comprising an endless belt 52 which passes around a pair of spaced apart, driven rollers 54,56.

The apparatus of FIG. 6 comprises an undriven let-off roll 72 which carries a roll of a second air permeable facing fabric 74; an idler roller 98; an apparatus 76 (analogous to apparatus 36 in FIG. 5) for making a second web or layer 78 of melt blown polypropylene fibers; an undriven let-off roll 100 carrying the aforementioned laminate 49; an idler roller 101; an electrostatic charging unit 80 (analogous to electrostatic charging unit 40 in FIG. 5) comprising an upper discharge bar 82 and a lower discharge bar 84; an idler roller 99; a driven take up roll 98 for collecting the electrostatically charged four-layer laminate 110; and a conveyor means comprising an endless belt 92 passing around spaced apart, driven rollers 94,96.

Main body portion 110 of face mask 10 is a four-layer laminated structure comprising a first facing layer 34, a first individual layer 38 of melt blown polypropylene fibers, a second individual layer 78 of melt blown polypropylene fibers, and a second facing layer 74.

The above-mentioned four-layer laminate 110 is prepared as follows. Referring to FIG. 5, facing fabric 34, which is a print bonded nonwoven fabric comprising a blend of about 65% wood pulp and 35% staple length rayon fibers, was let off from roller 32 and passed under idler roller 58 onto conveyor belt 52. Facing fabric 34 was then conveyed, in the direction shown by the arrow in FIG. 5, under melt blowing apparatus 36 which deposited a layer or web 38 of melt blown polypropylene fibers onto its upper surface 35. The melt blown polypropylene fibers had diameters ranging from about 2.5 microns to about 5.5 microns. The average diameter of these fibers was about 4.2 microns. The fibers ranged from about 76.2 mm (3 inches) to about 127 mm (5 inches) in length. The two-layer laminate 47 comprising nonwoven facing layer 34 and melt blown layer 38 was then conveyed, at a rate of about 19.8 m (65 feet) per minute, between discharge bars 42,44 of electrostatic charging unit 40. The discharge bars were arranged so that their facing surfaces were about 38.1 mm (1½ inches) apart. The voltage between the two discharge bars, with laminate 47 therebetween, was maintained at 15-20 kv. The electrostatic charging process is disclosed generally in U.S. Patent No. 4,375,718, the teachings of which are incorporated herein by reference. After passing through the electrostatic charging unit 40, the electrostatically charged laminate, identified in FIG. 5 by numeral 49, was rolled up on take up roll 48 to await further processing.

Referring now to FIG. 6, the electrostatically charged laminate 49 comprising facing layer 34 and polypropylene melt blown layer 38 was mounted on let-off roll 100 in such fashion that when the laminate was unrolled, facing layer 34 was oriented upwardly and polypropylene melt blown filtration layer 38 was oriented downwardly. This mounting and orientation can be seen toward the middle portion of FIG. 6 of the drawings. Referring to the left-hand side of FIG. 6, it will be seen that a second roll of air permeable facing material 74 has been mounted on let-off roller 72. Facing material 74 comprised the same print bonded air permeable nonwoven fabric used for facing material 34. Facing layer 74 was led under idler roller 98 onto conveyor belt 92 so as to be conducted in the left to right direction indicated by the directional arrow in FIG. 6. Facing material 74 was passed under melt blowing apparatus 76 which deposited a layer or web 78 of melt blown polypropylene fibers onto the upper surface 75 thereof to form a two-ply laminate 87. The characteristics of melt blown filtration layer 78 were identical to those of melt blown layer 38, i.e. the fibers ranged in diameter from about 2.5 microns to about 5.5 microns, had an average diameter of about 4.2 microns, and ranged in length from about 76.2 to 127 mm (3 to 5 inches). Just after leaving the confines of conveyor belt 92, the upper surface of laminated structure 87, which comprised facing material 74 and melt blown layer 78, was brought into face-to-face contact with previously electrostatically charged laminate 49 as it was being led under idler roller 101 and conducted in the direction indicated by the directional arrow in FIG. 6. As will be seen by reference to FIG. 6, electrostatically charged laminate 49 was brought into contact with laminated material 87 in such manner that the melt blown layer 38 of laminate 49 was brought into face-to-face contact with melt blown layer 78 of laminated material 87.

After the electrostatically charged laminate 49 and laminate 87 were combined in the fashion just mentioned, the resulting four-layer laminate was conveyed, at a rate of about 19.8 m (65 feet) per minute, between the discharge bars 82,84 of electrostatic charging unit 80 where the four-layer laminate was electrostatically charged. The spacing of discharge bars 82,84 was the same as the spacing of discharge bars 42,44 in electrostatic charging unit 40. A voltage of 15-20 kv was maintained between discharge bars 82,84. The electrostatically charged four-layer laminate, identified by the numeral 110 at the far right-hand side of FIG. 6, was then rolled up on take up roller 98 to await conversion into face mask 10 shown in FIG. 1.

Still referring to FIG. 6, it will be understood that the four-layer electrostatically charged laminate 110 might, in a commercial production apparatus, have a width on the order of 1.22-1.52 m (4-5 feet). A processing station, bearing numeral 115 in FIG. 6, may be located either before or after electrostatic charging unit 80. Processing station 115 can be used to cut the laminated material into smaller width rolls corresponding to the width needed for individual face masks. If desired, heat sealing of the edges of the individual rolls of laminated material could also be accomplished at station 115.

Two four-layer electrostatically charged laminates 110 were prepared using the apparatus and procedure just described. A nonwoven fabric comprising a blend of about 65% wood pulp fibers and about 35% staple length rayon fibers was used as facing fabric 34 and facing fabric 74 as well as the facing fabrics for the control materials to be described hereinafter. The various layers of polypropylene melt blown filtration medium were the same in all the samples and controls to the extent that they comprised melt blown polypropylene fibers which had diameters ranging from 2.5 microns to about 5.5 microns, average diameters of about 4.2 microns, and lengths in the range of about 76.2 mm to 127 mm (3 to 5 inches). Laminates in accordance with the present invention comprised two individual layers of melt blown polypropylene fibers in face-to-face contact as previously described. Control laminates comprised a single layer of melt blown polypropylene fibers. The weights of the melt blown polypropylene layers used for the samples and controls are described hereinafter.

Sample 1 of electrostatically charged four-ply laminate 110 according to the present invention utilized a filtration medium comprising a first fibrous layer 34 and a second fibrous layer 74 each of which had a basis weight of 20.34 gm$^{-2}$ (0.6 ounces/yd$^2$), resulting in a total basis weight of 40.68 gm$^{-2}$ (1.2 ounce/yd$^2$). Sample 2, which was not an example of the present invention but which served as a test control for Sample 1 mentioned above, utilized a filtration medium comprising a single layer of melt blown polypropylene fibers having a basis weight of 40.68 gm$^{-2}$ (1.2 ounce/yd$^2$). A piece of the aforementioned nonwoven facing material was placed on each side of the single layer of melt blown fibers, after which the resulting three-layer control laminate material was electrostatically charged by passing it through electrostatic charging unit 40 of FIG. 5 under the same charging conditions used for charging laminate 47.

In Sample 3, which is another example of the electrostatically charged four-ply laminate 110 of the present invention, the filtration medium utilized a first layer 34 of melt blown polypropylene fibers and a second layer 74 of melt blown polypropylene fibers, each of said fibrous layers having a basis weight of 13.56 gm$^{-2}$ (0.4 ounce/yd$^2$), resulting in a total basis weight was 27.12 gm$^{-2}$ (0.8 ounce/yd$^2$).

Sample 4, which was not an embodiment of the present invention but served as a test control for Sample 3, used a single layer of melt blown polypropylene fibers having a basis weight of 27.12 gm$^{-2}$ (0.8 ounce/yd$^2$). After superposing a piece of the aforementioned air permeable facing material on each side of the single layer of melt blown polypropylene fibers, the resulting three-layer laminate was electrostatically charged by passing it through electrostatic charging unit 40 under the same charging conditions used to charge laminate 47.

Samples 1, 2, 3 and 4 were thereafter tested for the following properties:

A.    % Bacterial Filtration Efficiency (% BFE);

B.    % Particle Filtration Efficiency using 0.8 micron particles (PFE-1);

C.    % Particle Filtration Efficiency using 0.1 micron particles (PFE-2);

D.    % Blood Repellency Rating (BRR); and

E.    Differential Pressure (Δ-P).

The results of the test are set forth in Table 1 which follows:

## TABLE 1

| Sample No. | Filtration Medium | % BFE | % PFE-1 | % PFE-2 | Blood Repellency Rating | ΔP |
|---|---|---|---|---|---|---|
| 1. | Two layers– $20.34\text{gm}^{-2}$ $\left(0.6 \text{ oz/yd}^2\right)$ each; $40.68\text{gm}^{-2}\left(1.2 \text{ oz/yd}^2\right)$ total | 99.8 | 98.3 | 95.9 | 1 | 3.1 |
| 2. | One layer– $40.68\text{gm}^{-2}\left(1.2 \text{ oz/yd}^2\right)$ | 96 | 97.5 | 72.1 | 2 | 6.1 |
| 3. | Two layers– $13.56\text{gm}^{-2}\left(0.4 \text{ oz/yd}^2\right)$ each; $27.12\text{gm}^{-2}\left(0.8 \text{ oz/yd}^2\right)$ total | 99.5 | 98.0 | 95.1 | 1 | 2.71 |
| 4. | One layer– $27.12\text{gm}^{-2}\left(0.8 \text{ oz/yd}^2\right)$ | 98.7 | 97.8 | 86.9 | 3 | 2.95 |

In Table 1, increasingly higher values for filtration efficiencies (BFE; PFE-1 and PFE-2) indicate increasingly better filtration performance. The smaller the Blood Repellency Rating (BRR), the better the resistance of the tested material to penetration by blood. The smaller the Differential Pressure Value (ΔP) the easier it will be to breathe through the laminated material.

The data set forth in Table 1 show that laminated materials according to the teachings of the present invention (Samples 1 and 3) have better blood repellency, better filtration efficiencies, and better breathability (i.e. are easier to breathe through) than their respective control materials. The improved filtration efficiency provided by laminated materials in accordance with the present invention are particularly evident in the Particle Filtration Efficiency Test using 0.1 micron particles (PFE-2). Comparing the results for Samples 1 and 2, it is seen that when the filtration medium utilized two layers of melt blown fibers each having a basis weight of 20.34 gm$^{-2}$ (0.6 ounce/yd$^2$) for a total basis weight of 40.68 gm$^{-2}$ (1.2 ounce/yd$^2$) (Sample 1), much better 0.1 micron particle filtration efficiency (95.9%) was obtained than when the filtration medium utilized a single layer of melt blown fibers having an identical basis weight of 40.68 gm$^{-2}$ (1.2 ounce/yd$^2$)(PFE-2 = 72.1%). The same improvement is seen when comparing Samples 3 and 4. The filtration medium of Sample 3 utilized two layers of melt blown fibers in face-to-face contact, each of said layers having a basis weight of 13.56 gm$^{-2}$ (0.4 ounce/yd$^2$)for a total basis weight of 27.12 gm$^{-2}$ (0.8 ounce/yd$^2$). Although the single layer of melt blown fibers used for the filtration medium of Sample 4 had a basis weight of 27.12 gm$^{-2}$ (0.8 ounce/yd$^2$), which is equivalzent to the total basis weight provided by the two 13.56 gm$^{-2}$ (0.4 ounce/yd$^2$) melt blown layers utilized for the filtration medium of Sample 3,

Sample 4 has a considerably lower PFE-2 rating (86.9%) than Sample 3 (PFE-3 = 95.1%).

Referring to the Blood Repellency Ratings shown in Table 1, it will be seen that the use of two melt blown fibrous layers each having a basis weight of 20.34gm$^{-2}$ (0.6 ounce/yd$^2$) (Sample 1) provides better blood repellency than does the use of two melt blown fibrous layers each having a basis weight of 13.56gm$^{-2}$ (0.4 ounce/yd$^2$)(Sample 3). Thus, the four-layer laminate of Sample 1 would be preferred where higher resistance to blood penetration is desired in the face mask.

The tests used in evaluate Samples 1-4 are described below.

Blood Repellency Test

The Blood Repellency Rating (BRR) was determined according to the following test. A face mask comprising the filtration medium to be tested was tied onto a mannequin head in the general manner in which it would be worn in use. A glass syringe having a 15 gauge stainless steel needle was mounted with its point 305 millimeters (twelve (12) inches) from the face mask and with the mounted face mask turned 20 degrees to the longitudinal axis of the needle. A piece of blotter stock was positioned behind and in contact with the face mask. Bovine blood was loaded into the syringe. 1.4 milliliters of the blood was projected against the outer surface of the face mask at 120 mmHg pressure. Any blood which passed through the mask impinged on and was collected by the blotter stock positioned behind the mask. A rating of 1 was assigned where no blood appeared on the blotter. A rating of 2 was assigned where the surface area of the blood stain on the blotter was equal to or less than the area of a circle whose diameter is 3mm. A rating of 3 was assigned where the surface area of the blood stain on the blotter was greater than the area of a circle whose diameter is 3mm but equal to or less than the area of a circle whose diameter is 5mm. A rating of 4 was assigned where the surface area of the blood stain was greater than the area of a circle whose diameter is 5mm but equal to or less than the area of a circle whose diameter is 9mm. A rating of 5 was assigned where the area of the blood stain on the blotter was greater than the area of a circle whose diameter is 9mm.

Breathability Test

Differential Pressure tests were conducted to determine the ease with which air passes through the material under test. The test results give an indication of how easy (or difficult) it will be for the wearer to actually breathe through a face mask when it is being used. In order to conduct the test, the material to be tested (i.e. inner facing/filtration medium/outer facing) was mounted in flat form in a clamping device which exposed 5.06 cm$^2$ of sample surface area to an incoming stream of air flowing at 8 liters per minute (LPM). The air pressure was measured, in mm of water, on the air inflow and air outflow sides of the material being tested. The Differential Pressure, $\Delta P$, is calculated as follows:

$$\Delta P = \frac{(P_o - P_i) \times 2}{A}$$

where $P_o$ = pressure of air on outflow side of the test material;
where $P_i$ = pressure of air on inflow side of the test material; and
A = the area of sample exposed to the air flow.

Results are reported in mm H$_2$O/cm$^2$. The lower the $\Delta P$, the easier it will be to breathe through the filtration material and/or a mask made therefrom.

Particulate Filtration Efficiency Test (PFE-1)

Particulate filtration efficiency for particles 0.8 micron and larger (PFE-1) was determined by using the test apparatus and procedure disclosed in U.S. Patent 4,382,378, the teachings of which are incorporated herein by reference. In summary, this test procedure employs a combination of water and particulate matter to challenge the filtration medium and determine its particulate filtration efficiency. The test projects an aerosol of latex comprising polymer particles in water, in a dilute air stream through the filtration medium to be tested and then to an optical particle counter to count the particles that have penetrated the filtration medium. Before the air stream is directed to the particle counter, the water in the system is evaporated and removed from the air stream. The particle counter therefore counts only the solidified latex particles that have penetrated the filtration medium. In this type of test, the filtration medium is challenged by a combination of liquid and solid components which are said to more accurately duplicate breath exhaled through the nose and mouth of the wearer of the mask. The latex particles in their dried state are substantially spherical in configuration and have a diameter of approximately 0.8 micron. The amount of solidified latex particles reaching the particle counter with no test sample mounted in the test device is used as a control. % PFE is determined by the following equation:

$$\% \text{ PFE} = \frac{\text{Particles (Control)} - \text{Particles (Test)}}{\text{Particles (Control)}} \times 100$$

% PFE is determined by dividing the number of solidified latex particles which pass through the filtration medium under test by the number of solidified latex particles in the control and multiplying by 100.

Particulate Filtration Efficiency Test (PFE-2)

This test is performed in the same manner as the above-described PFE-1 Test except the particles in the latex have a diameter of approximately 0.1 micron and the aerosol is dried so that the 0.1 micron diameter particles are in a dried state as they impinge upon the material to be tested.

Bacterial Filtration Efficiency (BFE) Test

Face masks were tested for their Bacterial Filtration Efficiency (BFE) using a "Chicago" nebulizer and 6-stage, "viable particle" Andersen Sampler. A 24-hour culture of Staphylococcus aureus (S. aureus) was diluted in sterile 1.5% aqueous peptone solution to a concentration sufficient to achieve a delivery of 2200 ± 500 viable particles to the control plates of the Andersen Sampler. The culture suspension was pumped through the Chicago nebulizer into an aerosol chamber at a flow rate of 28.3 liters/min. and constant air pressure to form aerosol droplets of desired size. The aerosol droplets were conducted through the aerosol chamber, passed through the material to be tested and collected in the Andersen Sampler. The Andersen Sampler, which is a sieve sampler, contained six agar plates, each successive plate being covered by a stainless steel screen of progressively smaller pore size. The media used was soybean casein digest agar 1%. The aerosol droplets entering the Andersen Sampler impinge on the aforementioned agar plates which, after exposure to the aerosol droplets, are incubated at 37°C for 24-48 hours. The colonies formed by each bacteria laden droplet are counted. Colony counts from the Andersen Sampler plates were converted to probable hit values using the chart of Andersen. Controls were run under identical conditions but with no test sample mounted between the aerosol chamber and the Andersen Sampler. Bacteria Filtration Efficiency was calculated as a percent difference between test sample runs and the control runs without a test sample in place. % BFE was determined according to the following equation:

$$\% \text{ BFE } = \frac{\text{Colonies (Control)} - \text{Colonies (Test)}}{\text{Colonies (Control)}} \times 100$$

## Claims

1.  A method for making the body portion of a face mask, said body portion comprising a first air permeable facing layer (34), a filtration medium and a second air permeable facing layer (74), said method comprising:
    a) conveying said first facing layer (34) in a first direction;
    b) depositing a first layer (38) of melt blown thermoplastic fibers on one surface of said first facing layer (34) to form a first laminate (47); and
    c) electrostatically charging said first laminate (47);
    characterized by:
    d) conveying said second facing layer (74) in a first direction;
    e) depositing a second layer (78) of melt blown thermoplastic fibers on one surface of said second facing layer (74) to form a second laminate (87);
    f) superposing said first laminate (47) on said second laminate (87) to provide a third laminate in which said first layer (38) of melt blown fibers and said second layer (78) of melt blown fibers are in face-to-face relationship; and
    g) electrostatically charging said third laminate.

2.  The method of claim 1, wherein said first laminate (47) and said third laminate are electrostatically charged at a range of 15 kilovolts to 20 kilovolts.

3.  The method of claim 1 or claim 2, wherein the fibers comprising said first (38) and second (78) layers of melt blown thermoplastic fibers are polypropylene or nylon fibers.

4.  The method of claim 3, wherein the fibers comprising said first (38) and second (78) layers of melt blown thermoplastic fibers are polypropylene fibers.

5. The method of any one of claims 1 to 4, wherein the fibers comprising said first (38) and second (78) layers of melt blown thermoplastic fibers have diameters ranging up to 10 μm.

6. The method of claim 5, wherein said fibers have diameters ranging from 2 μm to 6 μm.

7. The method of claim 6, wherein said fibers have diameters ranging from 2.5 μm to 5.5 μm.

8. The method of any one of claims 1 to 7, wherein the fibers comprising said first (38) and second (78) layers of melt blown thermoplastic fibers have lengths ranging from 76.2 mm to 127 mm (3 inches to 5 inches).

9. The method of any one of claims 1 to 8, wherein the fibers comprising said first (38) and second (78) layers of melt blown thermoplastic fibers are polypropylene fibers having diameters ranging from 2 μm to 6 μm and lengths ranging from 76.2 mm to 127 mm (3 inches to 5 inches), said first (38) and second (78) layers of polypropylene fibers each have basis weights ranging from 6.78 to 27.12 gm$^{-2}$ (0.2 ounce/yd$^2$ to 0.8 ounce/yd$^2$) and said first (38) and second (78) facing layers are nonwoven fabrics.

10. The method of claim 9, wherein said nonwoven fabrics (38,78) are a print bonded nonwoven fabric comprising a blend of about 65% by weight of woodpulp fibers and 35% by weight of rayon fibers.

## Patentansprüche

1. Verfahren zum Herstellen des Körperteiles einer Gesichtsmaske, wobei der Körperteil eine erste luftdurchlässige Decklage (34), ein Filtermedium und eine zweite luftdurchlässige Decklage (74) aufweist, wobei das Verfahren umfaßt:
   a) Fördern der ersten Decklage (34) in einer ersten Richtung;
   b) Ablegen einer ersten Lage (38) von schmelzgeblasenen thermoplastischen Fasern auf eine Fläche der ersten Decklage (34) zur Bildung eines ersten Laminates (41); und
   c) elektrostatisches Aufladen des ersten Laminates (41);
   gekennzeichnet durch:
   d) Zuführen der zweiten Decklage (34) in einer ersten Richtung;
   e) Ablegen einer zweiten Lage (78) von schmelzgeblasenen thermoplastischen Fasern auf eine Fläche der zweiten Decklage (74) zur Bildung eines zweiten Laminates (87);
   f) Übereinanderlegen des ersten Laminates (47) und des zweiten Laminates (87) zur Bildung eines dritten Laminates, bei welchem die erste Lage (38) von schmelzgeblasenen Fasern und die zweite Lage (78) von schmelzgeblasenen Fasern einander gegenüberliegen und
   g) elektrostatisches Aufladen des dritten Laminates.

2. Verfahren nach Anspruch 1, bei welchem das erste Laminat (47) und das dritte Laminat in einem Bereich von 15 Kilovolt bis 20 Kilovolt elektrostatisch aufgeladen sind.

3. Verfahren nach Anspruch 1 oder 2, bei welchem die Fasern, welche die erste Lage (38) und die zweite Lage (78) von schmelzgeblasenen thermoplastischen Fasern bilden, Polypropylen oder Nylonfasern sind.

4. Verfahren nach Anspruch 3, bei welchem die Fasern, welche die erste Lage (38) und die zweite Lage (78) von schmelzgeblasenen thermoplastischen Fasern bilden, Polypropylenfasern sind.

5. Verfahren nach einem der Ansprüche 1 bis 4, bei welchem die Fasern, welche die erste Lage (38) und die zweite Lage (78) von schmelzgeblasenen thermoplastischen Fasern bilden, Durchmesser bis 10 μm haben.

6. Verfahren nach Anspruch 5, bei welchem die Fasern Durchmesser im Bereich von 2 μm bis 6 μm haben.

7. Verfahren nach Anspruch 6, bei welchem die Fasern Durchmesser im Bereich von 2,5 μm bis 5,5 μm haben.

8. Verfahren nach einem der Ansprüche 1 bis 7, bei welchem die Fasern, welche die erste Lage (38) und die zweite Lage (78) von schmelzgeblasenen thermoplastischen Fasern bilden, Längen im Bereich von 76,2 mm bis 127 mm (3 Zoll bis 5 Zoll) haben.

9. Verfahren nach einem der Ansprüche 1 bis 8, bei welchem die Fasern, welche die erste Lage (38) und

EP 0 391 725 B1

die zweite Lage (78) von schmelzgeblasenen thermoplastischen Fasern bilden, Polypropylenfasern mit Durchmessern im Bereich von 2 μm bis 6 μm und Längen im Bereich von 76,2 mm bis 127 mm (3 Zoll bis 5 Zoll) sind, wobei die erste Lage (38) und die zweite Lage (78) aus Polypropylenfasern jeweils Basisgewichte von 6,78 bis 27,12 gm$^{-2}$ (0,2 Unzen/yd$^{-2}$ bis 0,8 Unzen/yd$^{-2}$) haben und die erste Decklage (38) und die zweite Decklage (78) ungewebte Stoffe sind.

10. Verfahren nach Anspruch 9, bei welchem die ungewebten Stoffe (38, 78) prägeverbundene Stoffe sind, die ein Gemisch aus etwa 65 Gew.-% Holzpulpefasern und 35 Gew.-% Rayonfasern aufweisen.

**Revendications**

1. Procédé de fabrication de la partie principale d'un masque respiratoire, la partie principale comprenant une première couche de revêtement perméable à l'air (34), un milieu de filtration et une deuxième couche de revêtement perméable à l'air (74), le procédé comprenant :
   a) le transport de la première couche de revêtement (34) dans une première direction;
   b) le dépôt d'une première couche (38) de fibres thermoplastiques soufflées à l'état fondu sur une face de la première couche de revêtement (34) pour former une première structure multicouche (47), et
   c) la charge de manière électrostatique de la première structure multicouche (47),
   caractérisé par :
   d) le transport de la deuxième couche de revêtement (74) dans une première direction;
   e) le dépôt d'une deuxième couche (78) de fibres thermoplastiques soufflées à l'état fondu sur une face de la deuxième couche de revêtement (74) pour former une deuxième structure multicouche (87);
   f) la superposition de la première structure multicouche (47) sur la deuxième structure multicouche (87) pour fournir une troisième structure multicouche dans laquelle la première couche (38) de fibres soufflées à l'état fondu et la deuxième couche (78) de fibres soufflées à l'état fondu sont en relation de face à face, et
   g) la charge de manière électrostatique de la troisième structure multicouche.

2. Procédé suivant la revendication 1, dans lequel la première structure multicouche (47) et la troisième structure multicouche sont chargées de manière électrostatique dans un intervalle de 15 kV à 20 kV.

3. Procédé suivant la revendication 1 ou 2, dans lequel les fibres composant les première (38) et deuxième (78) couches de fibres thermoplastiques soufflées à l'état fondu sont des fibres de polypropylène ou de Nylon.

4. Procédé suivant la revendication 3, dans lequel les fibres composant les première (38) et deuxième (78) couches de fibres thermoplastiques soufflées à l'état fondu sont des fibres de polypropylène.

5. Procédé suivant l'une quelconque des revendications 1 à 4, dans lequel les fibres composant les première (38) et deuxième (78) couches de fibres thermoplastiques soufflées à l'état fondu ont un diamètre ayant jusqu'à 10 μm.

6. Procédé suivant la revendication 5, dans lequel les fibres ont un diamètre se situant de 2 μm à 6 μm.

7. Procédé suivant la revendication 6, dans lequel les fibres ont un diamètre se situant de 2,5 μm à 5,5 μm.

8. Procédé suivant l'une quelconque des revendications 1 à 7, dans lequel les fibres composant les première (38) et deuxième (78) couches de fibres thermoplastiques soufflées à l'état fondu ont une longueur se situant de 76,2 mm à 127 mm (3 pouces à 5 pouces).

9. Procédé suivant l'une quelconque des revendications 1 à 8, dans lequel les fibres composant les première (38) et deuxième (78) couches de fibres thermoplastiques soufflées à l'état fondu sont des fibres de polypropylène ayant un diamètre se situant de 2 μm à 6 μm et une longueur se situant de 76,2 mm à 127 mm (3 pouces à 5 pouces), les première (38) et deuxième (78) couches de fibres de polypropylène ont chacune un poids de base se situant de 6,78 à 27,12 g par m$^2$ (0,2 livre par yd$^2$ à 0,8 livre par yd$^2$) et les première (34) et deuxième (74) couches de revêtement sont des toiles non tissées.

10. Procédé suivant la revendication 9, dans lequel les toiles non tissées (34, 74) sont une toile non tissée liée imprimée comprenant 65% en poids de fibres de pulpe de bois et 35% en poids de fibres de rayonne.

FIG.1

FIG.2

# FIG.3

# FIG.4

FIG.5

FIG.6